# Europäisches Patentamt
# European Patent Office
# Office européen des brevets

(11) Publication number: **0 122 952**
**B1**

(12) # EUROPEAN PATENT SPECIFICATION

(45) Date of publication of patent specification: **28.10.87**

(21) Application number: **83103967.2**

(22) Date of filing: **22.04.83**

(51) Int. Cl.⁴: **A 61 B 5/00,** G 01 N 27/46,
G 01 N 27/28

(54) Apparatus for measuring gas partial pressure in living body.

(43) Date of publication of application:
**31.10.84 Bulletin 84/44**

(45) Publication of the grant of the patent:
**28.10.87 Bulletin 87/44**

(84) Designated Contracting States:
**DE FR GB**

(56) References cited:
WO-A-80/02795
DE-A-2 648 607
GB-A-1 505 343
US-A-4 176 031

JOURNAL OF PHYSICS E - SCIENTIFIC
INSTRUMENTS, vol. 14, no. 7, July 1981,
Dorking C.E.W. HAHN "Techniques for
measuring the partial pressures of gases in the
blood. Part II - in vivo measurements", pages
783-797

(73) Proprietor: MITSUBISHI RAYON CO. LTD.
3-19, Kyobashi 2-chome Chuo-Ku
Tokyo 104 (JP)

(72) Inventor: Ooe, Akihiko
118-425, Komatsuji
Komaki-shi (JP)
Inventor: Imaiida, Tetsuo
25-2, Mikagecho-2-chome
Chikusa-ku Nagoya (JP)
Inventor: Uchida, Teruyoshi
13, Aza Kobayashi
Obata Moriyama-ku Nagoya (JP)
Inventor: Kojima, Hirotaka
1204, Tamanocho
Kasugai-shi (JP)

(74) Representative: Lehn, Werner, Dipl.-Ing. et al
Hoffmann, Eitle & Partner Patentanwälte
Arabellastrasse 4
D-8000 München 81 (DE)

## Description

This invention relates to apparatus for measuring partial pressure of gas in a living body based on the principle of polarography comprising:

a gas partial pressure sensor including a biomedical electrode, the tip end surface of said biomedical electrode being covered with a membrane and a counter electrode for producing an output signal indicative of a polarographic current, at least said biomedical electrode being suitable for placing in said living body;

a temperature sensor for placing at a position suitable for producing an output signal indicative of temperature (t) of said living body; and

an operation unit connected to receive the values of the output signals of said gas partial pressure sensor and said temperature sensor and operable to correct the output signal ($I_t$) of said gas partial pressure sensor.

Such apparatus is known from the Journal of Physics E-Scientific Instruments, Vol. 14, 1981 at page 788. The apparatus of the present invention may be employed to measure the concentration of various kinds of gases existing in biological tissue and biological fluid, especially blood, the use of the apparatus being one technique adapted for quantitatively gathering biological conditions in the field of medical science. In particular, the present invention relates to apparatus for measuring a partial pressure in a living body on the basis of polarography.

Two major methods have hitherto been available for the gas concentration or partial pressure measurements.

A first method is based on the so-called spot measurement wherein blood is extracted from a living body and thereafter the gas concentration is measured through a chemical analysis process.

A second method is such that a small sensor is inserted into a living body and the gas concentration in terms of an electrical signal is measured.

Disadvantageously, the first method consumes much time until a result is obtained and besides, blood being in process of extraction from the living body tends to come into contact with ambient air, and gas exchange will be caused between the extracted blood and the ambient air with the result that a measured representative of a gas concentration in the living body tends to be inaccurate.

The second method, on the other hand, is excellent and can provide substantially instantaneous results, thus facilitating on-line monitoring of a living body condition. This method has therefore been proposed in various ways mainly on the basis of the polarographic process. This method utilizing an electrode reaction, however, faces a problem of temperature dependency in which current or voltage values obtained greatly depend on temperatures and accurate values cannot be obtained unless the living body is kept at a constant temperature.

For example, a sensor for measuring the oxygen concentration in liquid usually has a cathode made of a noble metal such as gold, platinum, silver or the like and an anode, such as an Ag/AgCl electrode. Oxygen is reduced at the surface of the cathode and a polarographic current which has a small current value due to the reducing reaction is measured. Since the current value varies with the oxygen concentration in the liquid and temperature of the liquid as well, it cannot be concluded that a varying current value directly indicates a variation in the oxygen concentration in the liquid. Accordingly, in order to determine the variation in the oxygen concentration in the liquid, the liquid must be kept at a constant temperature or the oxygen concentration must be corrected by measuring temperature changes so as to be converted into a value at a reference temperature.

With an object to be examined in the form of a living body, however, it is practically difficult to maintain the living body at a constant temperature and such a maintenance is contradictory to the very purpose of on-line monitoring of the living body. For these reasons, appropriate temperature correction is required.

Further, in measuring a partial pressure of gas prevailing in blood with an electrode made of noble metal alone, measured values vary on account of pulsation or lowered reaction rate by blood component deposition on the electrode surface. To cope with such problems, it has been proposed to apply various coverings or coatings on the electrode surface. Since in this case the covering or coating by itself increases the temperature dependency, the need for temperature correction becomes more and more urgent.

Although the article from Journal of Physics E-Scientific Instruments mentioned above discusses the use of a thermocouple, in the tip of an *in vivo* intravascular electrode, for use in stabilizing temperature using an operational amplifier arrangement, it is stated that this proposal has not been generally adopted.

It can be clearly seen from a graph illustrating the known technique that the sensor output is not fully compensated and some variation occurs with temperature. This is in spite of the fact that compensation was attempted only over a relatively narrow temperature range of about 8°C.

No suggestion is made of how either to improve the compensation effect or to extend the range over which compensation is possible.

DE—A—26 48 607 (GB—A—1 517 034) proposed a probe for *in vitro* testing of samples which have, in the preferred embodiment, a thermistor connected to an operational amplifier for compensation purposes. In fact, the thermistor is intended to compensate both for permeability changes of the membrane and for changes in the solubility of oxygen with temperature. It is therefore clear that the compensation can only be approximate and not suitable for use over a wide range of temperatures. It is true that use of a microprocessor is suggested, this device to be pre-programmed with necessary correction factors at various temperatures. It is not quite clear what is envisaged, but there is certainly no suggestion of supplying the output of the temperature sensor to the microprocessor or of any special compensation rule. Furthermore, no suggestion is made of how to extend the range of temperatures over which accurate compensation may be achieved. In addition, there is no suggestion of applying the proposed techniques to *in vivo* testing.

It is an object of the present invention to provide apparatus which can achieve accurate and rapid measurements of a gas partial pressure in a living body on the basis of the polarography principle by compensating for temperature changes in the living body, over a relatively wide range of temperatures.

According to the invention, the apparatus defined in the first paragraph of this specification is characterized in that said pressure sensor and said temperature sensor are connected to an interface unit having an AD converter for digitizing the output signals from said partial pressure sensor and said temperature sensor, in that said operation unit is connected to said interface unit to receive digitized values of said output signals, in that said operation unit is arranged to correct the digitized output signal ($I_t$) of said pressure sensor to an output signal value of a reference temperature (T) in accordance with the following equation:

$$I_t = I_{sT}(k)^{T-t}$$

where $I_t$ is the output value of the gas partial pressure sensor at t°C, $I_{sT}$ is the output value of the gas partial pressure sensor at T°C, $k$ is the temperature coefficient of the gas partial pressure sensor, and $t$ is the temperature measured by the temperature sensor, and in that said operation unit is arranged to calculate a gas partial pressure on the basis of stored data representing a working curve prepared in said operation unit in advance, said working curve representing a relationship between said gas partial pressure and said corrected digitized output signal of said gas partial pressure sensor.

The gas partial pressure in the living body is usually represented by a value at a reference temperature of about 37°C. But sometimes the bodily temperature decreases by scores of centigrade degrees during a surgical operation. Especially during an operation for heart disease, the body is sometimes cooled to below 10°C. After the operation, the patient often becomes feverish with his temperature rising to over 40°C. Thus, the body temperature deviates from the reference temperature by over 10°C during the operation and by 5 to 6°C after the operation. Even when the dropping mercury electrode described previously, the electrolytic current varies by 2% as the temperature varies by 1°C and with the type of electrode used in the present invention, the electrolytic current varies by about 3%. For example, when the temperature of the living body deviates from the reference temperature by 20°C, the electrolytic current deviates from the actual value by 40 to 50%. Accordingly, without the correction according to the present invention, the measured value of the partial pressure in the living body greatly differs from an actual partial pressure. Conversely, the method of the present invention can reduce the error to a large extent to assure accurate measurement of actual gas partial pressure and can constantly apprise physicians and nurses of an accurate partial pressure in blood or tissue of the patient.

The invention will now be described by way of example with reference to the accompanying drawings.

Fig. 1 is a diagram useful in explaining the principle of polarography.

Fig. 2 is another diagram similar to Fig. 1 in which a platinum electrode has a porous membrane as a covering.

Fig. 3 is a circuit block diagram of the apparatus according to the present invention.

Fig. 4 is a detailed block diagram of the apparatus according to the present invention.

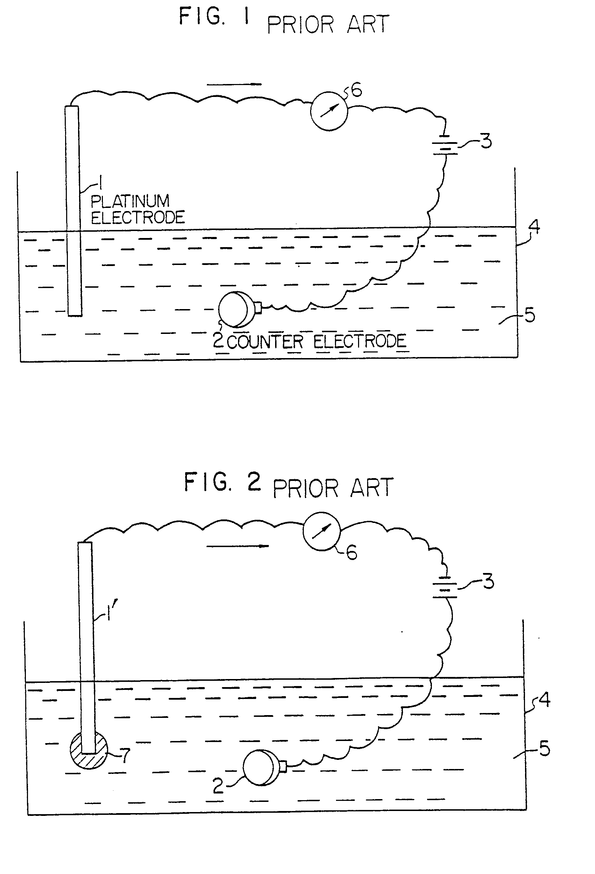

Referring to Fig. 1, when a voltage is applied across a cathode, or platinum electrode 1 which constitutes a biomedical electrode, and an anode, or counter electrode 2 in a solution 5 in a container 4 from a power supply 3, a polarographic current I due to electrolytic reaction flows and is indicated by a meter 6. With a dropping mercury electrode, for example, the current I varies under the influence of a temperature dependency because of the gas diffusion by about 2% at 1°C. In other words, the amount of the temperature dependency is about 2%/°C.

It has been proposed as shown in Fig. 2 to use a biomedical electrode 1' whose tip end surface is covered with a membrane which has an outer thin, dense layer having fine pores of an average diameter of 2 nanometers (20 Å) to 0.7 µm and an inner porous layer, contiguous to and integral with the outer layer, having fine pores of an average diameter of 0.7 µm or more, European patent application No. 81/301723.3 (EP—A—0056178) assigned to the same assignee as the present application. When the electrode 1' having its tip end covered with such a porous membrane 7 is used, the temperature dependency is larger than that of the dropping mercury electrode since many factors such as pore size, surface affinity and thickness of the porous membrane 7 affect the gas diffusion to increase the temperature dependency, thereby adversely affecting the gas partial pressure measurement. Through various experiments, the present inventors found that the polarographic current I was in a certain relationship with temperatures of an object to be examined, and that temperature correction for the polarographic current can be accomplished by using this relationship.

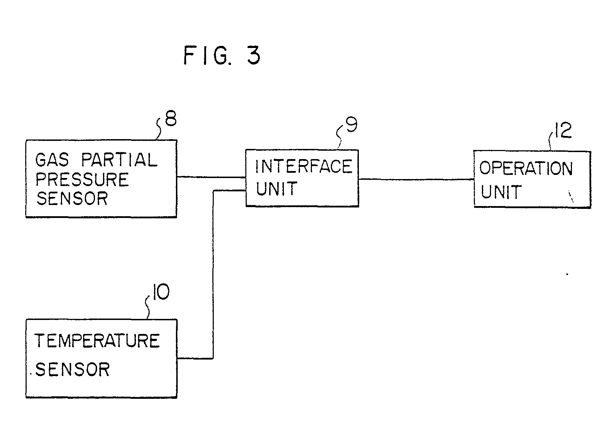

Fig. 3 is a block diagram for implementation of the present invention. In this embodiment, in order to eliminate the problems described previously, an operation unit 12 comprise a microcomputer board so that a value of a polarographic current I from a gas partial pressure sensor 8 is digitized by an interface unit 9 and inputted to the operation unit 12 and a biomedical temperature information signal from a temperature sensor 10 is digitized by the interface unit 9 and inputted to the operation unit 12, and the polarographic current value is temperature corrected and calculated from a predetermined value and a programmed formula to provide a gas partial pressure value. The same results may be obtained by an operation unit 12 of a mini-computer or of a dedicated logical current which substitutes for the microcomputer operation unit 12. A subsidiary advantage of the present invention resides in that in addition to the input data for a working curve preparation which is programmed in advance of measurement, a new value can be readily inputted at a desired time.

More particularly, in accordance with the principle of polarography, the output signal of the partial pressure sensor developing in the course of time lapse varies with not only temperatures but also, for example, amounts of substance in blood component deposited to the platinum electrode, or biomedical electrode as described previously, and variations in the sensor output signal can not be eliminated completely even when the porous membrane 7 is applied to the platinum electrode 1'. Accordingly, it is very advantageous that a new value for the working curve preparation can be inputted in an advanced phase of the measurement.

Correction procedures will now be described specifically.

As far as the principle of polarography is concerned, the linear relation, as represented by

$$P = aI + b \qquad (1)$$

where P is the gas partial pressure, I is the polarographic current value and $a$ and $b$ are constants, is held between the polarographic current value I and the gas partial pressure P. Accordingly, the gas partial pressure can be determined by measuring the polarographic current value whenever constants $a$ and $b$ are determined in advance. In this manner, a working curve which represents the equation (1) can be prepared.

The polarographic current I has a temperature dependency as described previously. The present inventors have studied the temperature dependency in various manners to find that the following equation holds approximately between the current value and the temperature:

$$I_t = I_{sT} k^{T-t} \qquad (2)$$

where $I_t$ is the polarographic current value at a temperature $t$, $I_{sT}$ is the polarographic value at a reference temperature T, and $k$ is the temperature coefficient. The temperature coefficient $k$ is specific to the gas partial pressure sensor but sensors produced under the same condition have substantially the same temperature coefficient. For precise measurement, however, several kinds of solutions having known gas partial pressure values are prepared, and currents and temperatures are measured for the respective kinds of solutions at different temperatures so that a temperature coefficient specific to a sensor participating in the measurement can be obtained through a method of least squares. For example, gas partial pressure sensors with platinum electrodes covered with the aforementioned porous membrane which were produced in the same lot under a certain condition had an average temperature coefficient $k$ of 0.97 with a variation of $\pm 0.01$. It follows therefore that if the manufacture condition is monitored and controlled properly, the temperature coefficient will satisfactorily be regarded as being constant for the purpose of the measurement.

Next, the relationship between gas partial pressure and current value, i.e., the working curve can be determined in various manners as itemized below.

(i) A biodmedical electrode of a gas partial pressure sensor is placed in an object to be measured and a temperature sensor is placed in or on the object to be measured. The working curve is then determined from a current value I and a temperature $t$ at this time, and a gas partial pressure value $P_{sT}$ in the object measured by using a separate instrument such as for example a commercially available batch type oxygen partial pressure measuring instrument, and a residual current value $I_0$ (current value for a gas partial pressure of zero) of the gas partial pressure sensor as well. More particularly, a current value $I_{sT}$ at a reference temperature T°C is determined from a current value $I_t$ measured at a temperature t°C in accordance with equation (2).

The corrected current value $I_{sT}$ thus determined is related to the gas partial pressure $P_{sT}$ in accordance with the equation (1). The constant $a$ in the equation (1) representative of a gradient of the current value relative to the gas partial pressure is $P_{sT}/(I_{sT}-I_0)$, and the constant $b$ which is P−al is determined by substituting $P_{sT}$, $P_{sT}/(I_{sT}-I_0)$, and $I_{sT}$ for P, $a$ and I, respectively, in equation (1).

(ii) A gas partial pressure sensor and a temperature measuring sensor are placed in one kind of solution having a known gas partial pressure value $P_{sT}$. The working curve is then determined from current value I, temperature $t$, gas partial pressure value P and residual current $I_0$ of the gas partial pressure sensor at this condition. Subsequently, constants $a$ and $b$ are determined as in item (i).

(iii) A gas partial pressure sensor and a temperature sensor are placed in two kinds of solutions having known gas partial pressure values, and the working curve is determined from current values $I_1$ and $I_2$, temperatures $t_1$ and $t_2$, and gas partial pressure values $P_1$ and $P_2$ for the different kinds of solutions in those conditions. In particular, the current value $I_1$ for one solution

and the current value $I_2$ for the other solution are respectively converted into current values at the reference temperature T°C as in items (i) and (ii) and the constants $a$ and $b$ in the equation (1) are determined in a similar manner.

The gas partial pressure P can be determined from the polarographic current value I in accordance with the equation (1), by using the constants $a$ and $b$ obtained through any one of methods itemized in (i), (ii) and (iii) as above.

The residual current $I_0$ in (i) and (ii) methods is specific to the gas partial pressure sensor. However, the present inventors have found that sensors produced under the same condition have substantially the same value of residual current in terms of a converted gas partial pressure, as well as the temperature coefficient $k$. For example, gas partial pressure sensors with platinum electrodes (biomedical electrodes) covered with the aforementioned porous membrane have a residual current which corresponds to an oxygen gas partial pressure of about 266 Pa (2 mmHg). As a result, the value of 266 Pa (2 mmHg) may be used as the value of $b$ instead of measuring the residual current $I_0$. The solution having a known gas partial pressure is required to contain electrolytic ions and preferably, it is physiological saline solution or blood.

The operator can select at will one of the above three methods. A fixed value of the temperature coefficient $k$ is inputted in advance but for precise measurement, the operator may input a value of the temperature coefficient $k$ specified to a sensor used.

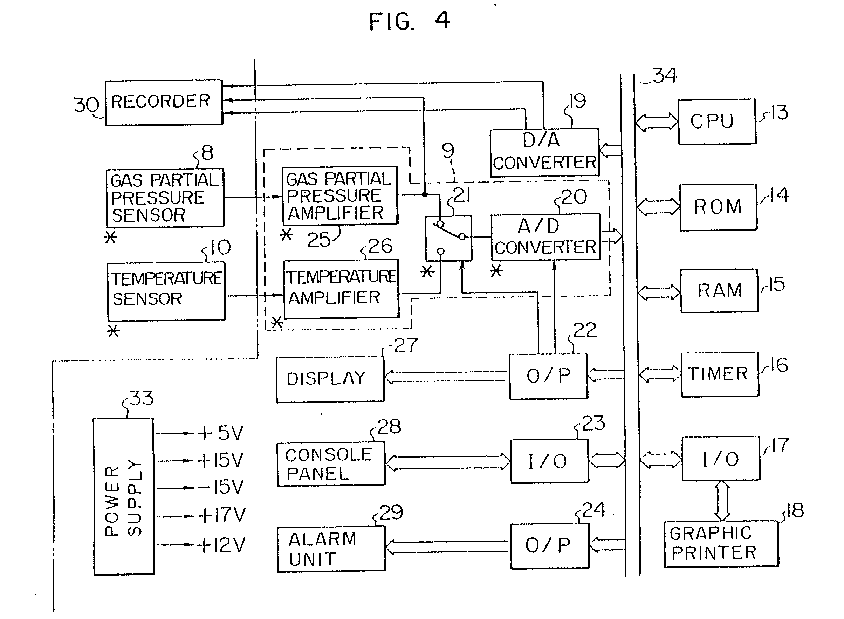

An arrangement for implementing the present invention will now be described with reference to Fig. 4.

In Fig. 4, an output signal from a gas partial pressure sensor 8 is amplified suitably by a partial pressure amplifier 25 and transferred to a CPU bus line 34 via a remote switch 21 and an A/D converter 20. Connected to the CPU bus line 34 are a CPU 13, a ROM 14, a RAM 15, a timer 16 and various I/O and O/P boards 17, 22, 23 and 24.

The output signal from the gas partial pressure sensor 8 transferred to the CPU bus 34 is stored in a pertinent area of the RAM 15 in accordance with a program which has been stored in the ROM 14 and by the action of the CPU 13. On the other hand, the output signal from the temperature sensor 10 is transferred to the CPU 13 via a temperature amplifier 26, the remote switch 21, the A/D converter 20 and CPU bus 34. The remote switch 21 is changed over at a suitable interval by a command via an O/P 22 in accordance with the program, for example, an interval of 200 to 1000 msec in a measurement of a partial pressure of oxygen in blood, and an interval of 5 to 10 sec in a measurement of a partial pressure in tissue in a living body, since in these cases generally, there is no need to recognize extremely rapid changes in the partial pressure and temperature. As a result, the output signals from the gas partial pressure sensor 8 and temperature sensor 10 are alternately and intermittently transferred to the CPU 13 depending on the interval of the change over of the remote switch 21.

In the CPU 13, a polarographic current value $I_t$ represented by the output signal of the gas partial pressure sensor 8 is corrected to a current value $I_{sT}$ at a reference temperature T as described in the foregoing, and then a gas partial pressure P at the reference temperature T is calculated on the basis of the corrected current value $I_{sT}$ and the working curve. The resultant gas partial pressure P calculated sequentially, or rather practically almost continuously, is displayed on a display 27 numerically and is recorded graphically and numerically on a graphic printer 18, together with the temperature value or the gas partial pressure alone.

An extension line may also be provided for delivery of the data to an ordinary analog recorder 30 where the recorder 30 is additionally used.

A console panel 28 includes a plurality of console panel switches adapted to input data for preparing the working curve before or during the calculation of the gas partial pressure and to input the operator instructions in order to effect an accurate and updated correction. The console panel 28 further includes pilot lamps (not shown) adapted to confirm or monitor the operations in progress.

The one series of data input system illustrated herein may be modified by doubling blocks marked with an asterisk in Fig. 4 (gas partial pressure sensor 8, temperature measuring sensor 10, remote switch 21, A/D converter, etc.). With this modification, oxygen partial pressure in arterial blood and that in a biological tissue inside the myocardium can be measured simultaneously. Results of the simultaneous measurements may be recorded on a single recorder 30 or graphic printer 18. If the results of the measurements exceed a predetermined control limit, audible or visual alarm unit 29 may be energized.

For production of the living body, especially, in the measurement of a human body, the amplifiers 25 and 26 may respectively include isolation amplifier circuits employing a transformer coupling. Further, other lines may also be isolated electrically.

As has been described, the apparatus of the present invention permits accurate and continuous monitoring of gas partial pressure in the living body and can be applied advantageously to clinical and experimental medical treatment.

**Claims**

1. An apparatus for measuring partial pressure of gas in a living body based on the principle of polarography comprising:

a gas partial pressure sensor (8) including a biomedical electrode (1, 1'), the tip end surface of said biomedical electrode being covered with a membrane (7) and a counter electrode (2) for producing an output signal indicative of a polarographic current, at least said biomedical electrode (1, 1') being suitable for placing in said living body;

a temperature sensor (10) for placing at a position suitable for producing an output signal indicative of temperature (t) of said living body; and

an operation unit (12) connected to receive the values of the output signals of said gas partial pressure sensor (8) and said temperature sensor (10), and operable to correct the output signal ($I_t$) of said gas partial pressure sensor (8),

characterized in that said pressure sensor (8) and said temperature sensor (10) are connected to an interface unit (9) having an A—D converter (20) for digitizing the output signals from said partial pressure sensor (8) and said temperature sensor (10), in that said operation unit (12) is connected to said interface unit to receive digitized values of said output signals, in that said operation unit is arranged to correct the digitized output signal ($I_t$) of said pressure sensor (8) to an output signal value at a reference temperature (T) in accordance with the following equation:

$$I_t = I_{sT}(k)^{T-t}$$

where $I_t$ is the output value of the gas partial pressure sensor at t°C, $I_{sT}$ is the output value of the gas partial pressure sensor at T°C, $k$ is the temperature coefficient of the gas partial pressure sensor, and $t$ is the temperature measured by the temperature sensor (10), and in that said operation unit (12) is arranged to calculate a gas partial pressure (P) on the basis of stored data representing a working curve prepared in said operation unit (12) in advance, said working curve representing a relationship between said gas partial pressure (P) and said corrected digitized output signal of said gas partial pressure sensor (8).

2. An apparatus according to claim 1 characterized by a display unit (27) for displaying the gas partial pressure calculated by said operation unit (12).

3. An apparatus according to claim 1 or 2 further characterized by an alarm unit (29) which is energized when the results of the calculation are beyond a predetermined control limit.

4. An apparatus according to any one of claims 1 to 3 characterized in that each of said gas partial pressure sensor (8) and tempereature sensor (10) is connected to an amplifier (25, 26) having an isolation amplifier circuit in said interface unit (9).

5. An apparatus according to any one of claims 1 to 4 further characterized by a graphic printer (18) for recording the gas partial pressure calculated by said operation unit (12).

6. An apparatus according to claim 5 further characterized by a second set of a gas partial pressure sensor (8), a temperature sensor (10), and an interface unit (9), whereby to form two sets of gas partial pressure measurement systems, and to display results of the two sets of measurement systems simultaneously on said single graphic printer (18).

7. An apparatus according to any one of claims 1 to 6 characterized in that said biomedical electrode (1') has a tip end surface covered with a membrane (7) comprising an outer dense, thin layer having fine pores of an average diameter of 2 nanometers (20Å) to 0.7 micrometers and an inner porous layer, contiguous to and integral with the outer layer, having fine pores of an average diameter of 0.7 micrometers or more.

**Patentansprüche**

1. Vorrichtung zum Messen des Partialdrucks eines Gases in einem lebenden Körper, basierend auf dem Prinzip der Polarographie, enthaltend:

einen Gaspartialdruck-Sensor (8) mit einer biomedizinischen Elektrode (1, 1'), wobei die Oberfläche des spitzen Endes der biomedizinischen Elektrode mit einer Membran (7) bedeckt ist, und einer Gegenelektrode (2) zum Erzeugen eines Ausgangssignals, das einen polarographischen Strom darstellt, wobei wenigstens die biomedizinische Elektrode (1, 1') zum Einsetzen in den lebenden Körper geeignet ist;

einen Temperatursensor (10), der an einer Stelle angeordnet ist, die zum Erzeugen eines Ausgangssignals geeignet ist, das die Temperatur (t) des lebenden Körpers darstellt und

eine Betätigungseinheit (12), die zum Empfangen der Größen der Ausgangssignale des Gaspartialdruck-Sensors (8) und des Temperatursensors (10) geschaltet ist, und zum Korrigieren des Ausgangssignals ($I_t$) des Gaspartialdruck-Sensors (8) betätigbar ist,

dadurch gekennzeichnet, daß der Drucksensor (8) und der Temperatursensor (10) mit einer Schnittstelleneinheit (9) mit einem A—D Wandler (20) zum Digitalisieren der Ausgangssignale aus dem Partialdruck-Sensor (8) und dem Temperatursensor (10) verbunden sind, daß die Betätigungseinheit (12) mit der Schnittstelleneinheit zum Empfangen von digitalisierten Größen der Ausgangssignale verbunden ist, daß die Betätigungseinheit zum Korrigieren des digitalisierten Ausgangssignals ($I_t$) des Drucksensors (8) in eine Ausgangssignalgröße bei einer Bezugstemperatur (T) entsprechend der folgenden Gleichung:

$$I_t = I_{sT}(k)^{T-t}$$

ausgebildet ist, wobei $I_t$ die Ausgangsgröße des Gaspartialdruck-Sensors bie t°C, $I_{sT}$ die Ausgangsgröße des Gaspartialdruck-Sensors bei T°C, $k$ der Temperatur-Koeffizient des Gaspartialdruck-Sensors und $t$ die durch den Temperatursensor (10) gemessene Temperatur ist, und daß die Betätigungseinheit (12) zum Berechnen eines Gaspartialdrucks (P) basierend auf gespeicherten Daten ausgebildet ist, die eine vorher in der Betätigungseinheit (12) vorbereitete Arbeitskurve darstellt, wobei die Arbeitskurve eine Beziehung zwischen dem Gaspartialdruck (P) und dem korrigierten digitalisierten Ausgangssignal des Gaspartialdruck-Sensors (8) darstellt.

2. Vorrichtung nach Anspruch 1, gekennzeichnet durch eine Anzeigeeinheit (27) zum Anzeigen des durch die Betätigungseinheit (12) berechneten Gaspartialdrucks.

3. Vorrichtung nach Anspruch 1 oder 2, weiterhin gekennzeichnet durch eine Alarmeinheit (29), die erregt wird, wenn die Ergebnisse der Rechnung oberhalb eines vorbestimmten Kontrollgrenzmaßes liegen.

4. Vorrichtung nach einem der Ansprüche 1 bis 3, dadurch gekennzeichnet, daß sowohl der Gaspartialdruck-Sensor (8) als auch der Temperatursensor (10) mit einem Verstärker (25, 26) mit einer Trenn-Verstärkungsschaltung in der Schnittstelleneinheit (9) verbunden sind.

5. Vorrichtung nach einem der Ansprüche 1 bis 4, weiterhin gekennzeichnet durch einen graphischen Drucker (18) zum Aufzeichnen des durch die Betätigungseinheit (12) berechneten Gaspartialdrucks.

6. Vorrichtung nach Anspruch 5, weiterhin gekennzeichnet durch einen zweiten Satz eines Gaspartialdruck-Sensors (8), eines Temperatursensors (10) und einer Schnittstelleneinheit (9), um hierdurch zwei Sätze Gaspartialdruck-Messungssysteme zu bilden, und um die Ergebnisse der beiden Meß-System-Sätze gleichzeitig auf dem einzigen graphischen Drucker (18) anzuzeigen.

7. Vorrichtung nach einem der Ansprüche 1 bis 6, dadurch gekennzeichnet, daß die biomedizinische Elektrode (1') eine mit einem spitzen Ende versehene Oberfläche aufweist, die mit einer Membran (7) bedeckt ist, die eine äußerliche dichte, dünne Schicht mit feinen Poren eines durchschnittlichen Durchmessers von 2 nanometer (20 Å) bis 0,7 mikrometer und eine innere poröse Schicht mit feinen Poren eines durchschnittlichen Durchmessers von 0,7 mikrometer oder mehr enthält, die an die äußere Schicht angrenzt und mit dieser integriert ist.

**Revendications**

1. Un appareil de mesure de pression gazeuse partielle dans un corps vivant basé sur le principe de la polarographie comprenant:

un capteur de pression gazeuse partielle (8) comportant une électrode biomédicale (1, 1'), la surface de l'extrémité distale de ladite électrode biomédicale étant couverte par une membrane (7), et une contre-électrode (2) pour fournir un signal de sortie représentatif d'un courant polarographique, ladite électrode biomédicale (1, 1') au moins étant adaptée à être placée dans ledit corps vivant;

un capteur de température (10) destiné à être placé en une position propre à lui permettre de fournir un signal de sortie représentatif de la température (t) dudit corps vivant; et

une unité d'exploitation (12) connectée de manière à recevoir les valeurs des signaux de sortie dudit capteur de pression gazeuse partielle (8) et dudit capteur de température (10), et adaptée à corriger le signal de sortie ($I_t$) dudit capteur de pression gazeuse partielle (8),

caractérisé en ce que ledit capteur de pression (8) et ledit capteur de température (10) sont reliés à une unité d'interface (9) comportant un convertisseur analogique-numérique (20) destiné à numériser les signaux de sortie dudit capteur de pression partielle (8) et dudit capteur de température (10), en ce que ladite unité d'exploitation (12) est reliée à ladite unité d'interface de manière à recevoir des valeurs numérisées desdits signaux de sortie, en ce que ladite unité d'exploitation est agencée de manière à corriger le signal de sortie numérisé ($I_t$) dudit capteur de pression (8) en le ramenant à une valeur de signal de sortie à une température de référence (T) conformément à l'équation:

$$I_t = I_{sT}(k)^{T-t}$$

où $I_t$ est la valeur de sortie du capteur de pression gazeuse partielle à t°C, $I_{sT}$ est la valeur de sortie du capteur de pression gazeuse partielle à T°C, $k$ est le coefficient de température du capteur de pression gazeuse partielle et $t$ est la température mesurée par le capteur de température (10), et en ce que ladite unité d'exploitation (12) est agencée de manière à calculer une pression gazeuse partielle (P) d'après des données en mémoire représentant une courbe de travail préparée par avance dans ladite unité d'exploitation (12), ladite courbe de travail représentant une relation entre ladite pression gazeuse partielle (P) et ledit signal de sortie numérisé et corrigé dudit capteur de pression gazeuse partielle (8).

2. Un appareil selon la revendication 1, caractérisé par une unité de visualisation (27) destinée à visualiser la pression gazeuse partielle calculée par ladite unité d'exploitation (12).

3. Un appareil selon la revendication 1 ou 2, caractérisé en outre par un avertisseur (29) qui est déclenché lorsque les résultats du calcul sont au-delà d'une limite de commande prédéterminée.

4. Un appareil selon l'une quelconque des revendications 1 à 3, caractérisé en ce que chacun desdits capteurs de pression gazeuse partielle (8) et de température (10) est relié à un amplificateur (25, 26) comportant un circuit amplificateur d'isolement dans ladite unité d'interface (9).

5. Un appareil selon l'une quelconque des revendications 1 à 4, caractérisé en outre par une imprimante graphique (18) destinée à enregistrer la pression gazeuse partielle calculée par ladite unité d'exploitation (12).

6. Un appareil selon la revendication 5, caractérisé en outre par un deuxième ensemble à capteur de pression gazeuse partielle (8), capteur de température (10) et unité d'interface (9), pour former ainsi deux ensembles de systèmes de mesure de pression gazeuse partielle et permettre de visualiser simultanément des résultats des deux ensembles de systèmes de mesure sur une même imprimante graphique (18).

7. Un appareil selon l'une quelconque des revendications 1 à 6, caractérisé en ce que ladite électrode biomédicale (1') présente une surface d'extrémité distale couverte par une membrane (7) comprenant une couche externe mince et dense présentant des pores fins d'un diamètre moyen de 2 nanomètres (20 Å) à 0,7 micromètre et une couche interne poreuse, contiguë à et d'un seul tenant avec la couche externe, présentant des pores fins d'un diamètre moyen de 0,7 micromètre ou plus.

FIG. 1 PRIOR ART

PLATINUM
ELECTRODE

2 COUNTER ELECTRODE

FIG. 2 PRIOR ART

0 122 952

# FIG. 3

GAS PARTIAL PRESSURE SENSOR `8`

INTERFACE UNIT `9`

OPERATION UNIT `12`

TEMPERATURE SENSOR `10`

2

FIG. 4